# EUROPEAN PATENT APPLICATION

(11) **EP 3 403 724 A1**
(43) Date of publication of application: **21.11.2018**
(21) Application number: 17305580.7
(22) Date of filing: 18.05.2017
(51) Int. Cl.: B01L 3/00, C04B 35/624, C12Q 1/68

(54) **PROCESS FOR MANUFACTURING AN ARRAY WITH MICROCHANNELS**

(71) Applicant: HIFIBIO, 75005 Paris (FR)
(72) Inventor: REITZ, Arnaud, 78280 GUYANCOURT (FR); REICHEN, Marcel, CH-8820 WADENSWIL (CH); SHEN, Bingqing, 92320 CHATILLON (FR); ELLOUZE, Sami, 92290 CHATENAY MALABRY (FR)
(74) Representative: CH Kilger Anwaltspartnerschaft mbB

(57) **Abstract**

The invention relates to a process for manufacturing a microfluidic chip comprising a solid material obtained from a sol-gel solution, the process comprising successively:
a) casting a sol-gel solution made with tetraethyl orthosilicate onto a mold presenting a relief pattern and having a different thickness over the whole of the mold;
b) gelling the sol-gel solution;
c) unmolding and drying the gel obtained in b), so as to obtain a solid glass; and
d) bonding said solid glass to a support, so as to obtain the microfluidic chip.

## Description

The present invention relates to a process for manufacturing a microfluidic chip comprising solid glass obtained from a sol-gel solution, as well as to the obtained microfluidic chip and uses thereof.

Conventionally, microfluidic chips useful for cell sorting or for assessing biological reactions are made up of a silicone material, such as polydimethylsiloxane (PDMS). The mold used in the manufacturing process may also be made of such a flexible material (like PDMS).

Some microstructured objects, which may be convenient for the field of microfluidics, may be made by a sol-gel process using a PDMS mold; this is notably described in US9266765.

Classically, the sol-gel process consists in preparing a solution containing precursors based on metal or metalloid elements (which may be organometallic compounds or metal salts) and one or more organic solvents, the resulting solution thereby forming a sol (which may also be designated sol-gel solution). As the sol already comprises water, the precursors contained in this sol-gel solution undergo, in part, a step of hydrolysis and a step of condensation, to form an oxide network entrapping the solvent, so as to form a gel. The gel is then dried, to form at the end of said drying a monolithic object.

However, using PDMS for microfluidic chips has many drawbacks:
o enclosure of particles, e.g. dust, is not uncommon;
o it is not suitable for high throughput processes because of its unreliability (particularly for bonding). Without surface modification, it may also destabilize the introduced biological sample (in emulsion form), by making the droplets coalesce;
o it is time-consuming and allows a low scalability potential; and
o it induces droplets shrinkage over time.

On the other hand, thermoplastics materials also have a low compatibility with solvents. Besides, the surface quality is extremely dependent on the micromachining steps, which leave surface roughness that may induce a deterioration of emulsions injected therein. They are often incompatible with cleansing methods used for glass. Finally, it may be difficult to perform an efficient bonding of a piezoelectric material on such materials.

There is thus a need for a process for manufacturing a microfluidic chip, which would be quick and easy to perform, at a high scalability. Moreover, there is a need for manufacturing a microfluidic chip in a stable material, suitable for high throughput processes, and which is less prone to particle enclosure. Said microfluidic chip would have to be usable at an industrial scale, and be in a material compatible with a bonding of a piezoelectric material. It would have to be compatible with cleansing methods used for glass, and preserve a good quality of the emulsions injected therein.

The inventors have discovered a specific process for manufacturing a solid glass microfluidic chip obtained from a sol-gel solution. Said solid glass microfluidic chip may be particularly used for screening immune cells such as antibody-producing cells, T or NK cells, or neutrophils.

Said process is suitable for high throughput screening, and the material in use is stable and reduces coalescence or droplet breaking. By "stable", it is meant that the used material is chemically and mechanically stable. Indeed, it is not degraded over time; besides, it may be washed properly many times in order to be reused, without contamination risk. Finally, it allows a long-term storage of the chips, particularly of the chips comprising biological samples, by avoiding pervaporation of water (thus avoiding the induction of droplets shrinkage) or oil comprised in said samples.

### SUMMARY OF THE INVENTION

The present invention thus relates to a process for manufacturing a microfluidic chip comprising a solid material obtained from a sol-gel solution, the process comprising successively:
a) casting a sol-gel solution made with tetraethyl orthosilicate onto a mold presenting a relief pattern and having a different thickness over the whole of the mold;
b) gelling the sol-gel solution;
c) unmolding and drying the gel obtained in b), so as to obtain a solid glass; and
d) bonding said solid glass to a support, so as to obtain the microfluidic chip.

The invention also relates to a microfluidic chip obtainable by the process according to the invention.

The invention further provides for the use of a microfluidic chip obtainable by the process according to the invention for screening proteins, preferably antibodies.

The invention also relates to a process for screening cells producing a protein of interest, preferably immune cells, comprising the following steps:
- introducing emulsion droplets comprising cells producing a protein, preferably immune cells and more preferably antibody- or cytokine-secreting cells, and either beads coated with capture agents, or cells expressing capture agents at their surface or within the cell, into a microfluidic chip of the present invention;
- incubating the resulting chip under conditions allowing protein secretion; and
- sorting cells producing the protein of interest based on a specific optical signal.

Said signal may be fluorescent or luminescent. It may be detected for example after a laser excitation, with a suitable bandpass filter such as a photomultiplier or an avalanche photodiode.

Such a process is for sorting cells, preferably immune cells, and is also called the "cell sorter process" or "cell sorting process".

Preferably, each emulsion droplet comprises (i) a single cell, preferably a single immune cell, and (ii) a single bead coated with capture agents or a suspension of magnetic beads coated with capture agents, or a single cell expressing capture agents.

The present invention also relates to a process for identifying nucleic acid sequences of interest, comprising the following steps:
- introducing emulsion droplets comprising cells into a microfluidic chip of the present invention, wherein each droplet comprises a single cell;
- putting a second droplet onto each emulsion droplet, wherein the second droplet comprises a defined number of beads coated with capture agents, preferably a single bead, and a reagents mixture, so that a fusion between each second droplet and each emulsion droplet is obtained, and then nucleic acid sequences of interest are delivered in said fusion; and
- capturing and optionally barcoding the nucleic acid sequences of interest,
- and then optionally recovering the fused droplets.

Such a process is also called the "barcoding process".

### DESCRIPTION OF THE INVENTION

The process for manufacturing a microfluidic chip comprising a solid material obtained from a sol-gel solution according to the invention comprises successively:
a) casting a sol-gel solution made with tetraethyl orthosilicate onto a mold presenting a relief pattern and having a different thickness over the whole of the mold;
b) gelling the sol-gel solution;
c) unmolding and drying the gel obtained in b), so as to obtain a solid glass; and
d) bonding said solid glass to a support, so as to obtain the microfluidic chip.

A microfluidic chip comprises a substrate and a support, defining together at least a channel. By "microfluidic", it is generally meant that the transversal dimensions of the channel in which the droplets or fluid are intended to circulate are smaller than one millimeter and are comprised for example between 1 µm and 1 mm.

The substrate of the microfluidic chip corresponds, in the present invention, to the solid material obtained from a sol-gel solution, i.e. to a solid glass. The support of the microfluidic chip is preferably a glass slide or a wafer.

A microfluidic chip is generally a block containing channels with rectangular, elliptic or circular cross-sections. The thickness of a microfluidic chip is generally less than 1 cm.

The channel is delimited by side walls defined in the substrate or in the support. Preferably, the side walls are defined in the substrate. Indeed, in this case, the relief pattern of the mold used in step a) of the process described above, thus forming the substrate of the microfluidic chip, comprises protrusions, so that the gelled sol-gel solution of step b) comprises channels, such as microchannels or nanochannels.

The channel is elongated in a main direction.

The channel presents a height and a width, measured in a plan transversal to the main direction. The height and/or the width are smaller than 1 mm, preferably comprised between 1 µm and 1 mm.

For example, the channel presents a square or a rectangular section in the plan transversal to the main direction. As an alternative, the channel presents a circular or elliptical section in the plan transversal to the main direction.

The channel usually comprises an inlet to inject fluid and an outlet to collect fluid. The channel is usually intended to allow flowing of a fluid along the main direction from the inlet toward the outlet.

The inlet of the channel can be connected to a reservoir of a fluid intended to flow in the channel. The outlet of the channel can be connected to a reservoir to collect the fluid coming from the channel.

A microfluidic chip may comprise a plurality of channels, and comprises junctions between the channels.

The junction can be divergent, so that a fluid arriving in a first channel separates in two different channels after the junction.

In alternative a junction can be convergent such that two fluids arrive from two different channels.

The junctions are, for example, T junctions or flow-focusing junctions.

For example, junctions are used to help the generation of droplets of an inner fluid in a carrier fluid, the carrier fluid being immiscible with the inner fluid.

The microfluidic chip may further comprise a filtration unit.

The microfluidic chip may further comprise an incubation chamber.

A microfluidic chip is typically intended to prepare successive droplets of an inner fluid, dispersed in an outer fluid. The droplets comprise at least one medium dispersed or dissolved in the inner fluid, preferably at least a first medium dispersed and/or dissolved in the inner fluid, and a second medium dispersed and/or dissolved in the inner fluid.

A microfluidic chip may comprise one or more of the following feature(s), taken solely, or according to any technical possible combination:
- a supply zone for introducing a fluid;
- a collection zone for collecting a fluid; and
- a working channel extending in a main direction.

The microfluidic chip is preferably used as a biological assay system which delivers droplets each containing a single biological entity and at least a medium able to interact with the single biological entity. An example of single biological entity is a single cell, such as a single immune cell, particularly a single antibody-producing cell or a single cytokine-producing cell. The cell may be a prokaryotic (i.e. bacterial) cell, or a eukaryotic cell. By "immune cell", it is meant an antibody-producing cell (i.e. a B cell), or a cytokine-producing cell such as a T cell, an NK cell, or a neutrophil. Said single cell may be lysed or not lysed. In the cell sorter process, a cell producing a protein is typically isolated in a droplet of first fluid with a medium comprising a buffer. In a second fluid, a second medium comprising beads coated or grafted with capture agents, or cells expressing capture agents on their membrane surface or within the cell, is present. The first fluid and the second fluid are mixed, and typically added to a third fluid (such as a lipophilic or a fluorophilic fluid), to form the emulsion droplets. The fluorophilic fluid (third fluid) may be HFE fluorinated oils from 3M Novec.

In the barcoding process, the microfluidic chip preferably comprises a chamber with an array of capture systems allowing droplets holding in place. It typically comprises an inlet and an outlet.

The beads used in the cell sorter process and in the barcoding process may be magnetic beads. Magnetic beads may be made of iron or any other magnetic material. Magnetic beads are useful because they can concentrate the signal upon magnetic excitation, i.e. they can form a line which is easily detectable. Alternatively, they may be hydrogel beads.

The cell sorter process may use cells expressing capture agents instead of beads coated with capture agents, also called reporter cells. Said cells may express capture agents at their surface, or may express capture agents within said cell. They may also be cells labeled with a fluorescent capture agent such as a fluorescent antibody. Such cells may be prokaryote or eukaryote cells. Prokaryote cells are notably bacterial cells. They may be wild-type, or engineered cells. By engineered, it is meant that the wild-type cell is modified by artificial genetic mutations. An engineered cell may be a GMO (Gene Modified Organism). The reporter cells are preferably CHO cells, such as CHO-S cells.

Capture agents include but are not necessarily limited to antibodies, antibody derivatives (such as scFv) and antigen binding fragments thereof, proteins, metabolites, as well as nucleic acid sequences. In some embodiments, the beads are functionalized so that they can be covalently or non-covalently associated with capture agents. In some embodiments, notably for the cell sorting process, the beads comprise, for example, streptavidin covalently coupled to the surface of the beads, and as such are configured to form complexes with a biotinylated component, such as a biotinylated antibody or antigen binding fragment thereof. In another embodiment, the beads are coated with a monolayer of streptavidin. In some embodiments the beads comprise functionalized groups on their surfaces, or chemical groups that are capable of being functionalized. In one embodiment the beads are made of at least one organic polymer, which is able to form a hydrogel in water. Said polymer may comprise acrylamide, polyethylene glycol or acrylate monomers. In some embodiments, the beads can be hydrophilic, and positively charged or negatively charged. In embodiments, the beads can be provided pre-loaded with one or more capture agents onto their surfaces so that they can be used with the microfluidic chips to capture the proteins of interest such as antibodies and/or their sequences for which the one or more capture agents are specific.

Preferably, in the cell sorting process, the capture agents are antibodies, antibody derivatives or antigen binding fragments thereof.

Preferably, in the barcoding process, the capture agents are nucleic acid sequences. By nucleic acid sequences, it is meant DNA (including cDNA) or RNA. Preferably, the capture agents are DNA oligonucleotides, such as DNA oligonucleotides comprising from 2 to 40 nucleotides (nt), preferably from 5 to 25 nucleotides as capturing part. They are linked to functional DNA as barcode or specific DNA sequences used for sequencing and/or library amplification.

Typically, the DNA oligonucleotides which may be used have the following structure:

(L)-(T7)-(Sbs12/Sbs3)-(BCD)-(GSPs)

wherein :
**L** is a linker allowing cleavage of the barcode sequence, for example under light exposure. L may be present or not;
**T7** is an In Vitro Transcription (IVT) sequence, which may be present or not. It is used for IVT in some applications, or as a PCR primer;
**Sbs12 and Sbs3** are Illumina sequences. They are used for library preparation for Illumina sequencing platform. They allow reading of each DNA strand, starting from the sense strand or the antisense strand;
**BCD** is a barcode index used to generate the diversity of barcode builds, notably obtained from a composition comprising a defined number of different B, a defined number of different C and a defined number of different D.
This barcode sequence may be different as its length can vary and/or the number of indexes could vary (it could be ABCDEFG for example); and
**GSPs (Gene Specific Primer)** is a sequence used for mRNA capture. It may be a Reverse Transcription (RT) primer for capturing VH and VL sequences with the same barcode, but it may also be a more general primer. In such an example, each bead present in second droplets comprises a DNA oligonucleotide with the above structure in which BCD is a single barcode and GSPs is a fusion sequence between half of VH primer and half of VL primer.

Preferably, the barcoding process uses a mixture of reagents which is adapted to a molecular biology technique. Said molecular biology technique may be droplet Reverse Transcription (dRT), droplet Polymerase Chain Reaction (dPCR), dRT-PCR, quantitative droplet RT-PCR (qdRT-PCR), droplet In Vitro Transcription (dIVT), droplet DNA second strand synthesis (dDNA SSS), droplet Rolling Circle Amplification (dRCA). The mixture of reagents may comprise a primer set, which is preferably gene specific, sequence specific, but which may also be adapted for random priming or whole priming (such as « Full RNA-seq » based on Poly A tailing of certain mRNA).

According to a first embodiment, preferably, the microfluidic chip is a cell sorter. In such a case, it is adapted for the cell sorting process. Thus, when forming the emulsion droplets, the biological entities are put in contact with the beads coated or grafted with capture agents, or with the cells expressing said capture agents on their surface or within the cell. The relevant droplets (i.e. comprising the biological entities such as antibody-producing cells or cytokine-producing cells) are then identified based on binding of the biological entity or its product (such as the antibodies or cytokines produced) and the beads coated or grafted with capture agents, or the cells expressing said capture agents on their surface or within the cell. The single biological entity (such as the single antibody producing cell or the single cytokine producing cell) in each droplet can be recovered and further analyzed.

The cell sorter process according to the invention thus comprises the following steps:
- introducing emulsion droplets comprising cells producing a protein, preferably immune cells and more preferably antibody- or cytokine-producing cells, and either beads coated with capture agents or cells expressing capture agents on their surface or within the cell, said emulsion droplets being optionally previously incubated in bulk, into a microfluidic chip of the present invention; and
- sorting cells producing the protein of interest based on a specific optical signal.

By "previously incubated in bulk", it is meant that the first fluid comprising the cells (such as immune cells and preferably antibody- or cytokine producing cells) in a medium comprising a buffer, and the second fluid comprising beads coated or grafted with capture agents, or cells expressing capture agents on their membrane surface or within the cell, are mixed and incubated, after forming emulsion droplets. The first fluid and the second fluid are added to a third fluid (such as a lipophilic or a fluorophilic fluid), to form the emulsion droplets. Then, the incubation is performed. The incubation lasts for a time sufficient to allow binding of the produced protein to the capture agents, for example a few hours, for example from 1 h to 6h.

According to another embodiment, the cell sorter process according to the invention comprises the following steps:
- introducing emulsion droplets comprising cells producing a protein, preferably immune cells and more preferably antibody- or cytokine-producing cells, and either beads coated with capture agents or cells expressing capture agents on their surface or within the cell, into a microfluidic chip of the present invention;
- incubating the resulting chip under conditions allowing protein secretion; and
- sorting cells producing the protein of interest based on a specific optical signal.

According to a second embodiment, preferably, the microfluidic chip is an array used for isolating a nucleic acid sequence of interest, particularly some DNA sequences of interest. Said droplet array comprises wells, wherein each well comprises a droplet of a single biological entity. Said biological entity may be a cell, preferably an immune cell, preferably an antibody-producing cell.

Then, a second droplet comprising the beads, preferably comprising a single bead, and a reagents mixture, is put in contact with each of the wells. A fusion (i.e. mixture) between the droplet of biological entity and the droplet comprising the beads happens. By "fusion", it is meant that a droplet of biological entity is mixed with a second droplet (i.e. comprising the beads), in order to form a bigger unique resulting droplet. Said fusion may be obtained by applying a high voltage. The reagents mixture allows lysis of the biological entities, particularly of the cells of interest. Due to the reagents mixture, the fusion allows lysis of the biological entities, such as immune cells, and thereby linkage between the nucleic acid of interest, which is present in the biological entities, and the capture agents which are present on the beads. The relevant wells (i.e. comprising the biological entity such as cells of interest) are then identified due to the binding of the nucleic acid sequence of interest of the biological entity, and the beads coated or grafted with capture agents. Then, the single biological entity (such as the single antibody producing cell) in each droplet can be identified and recovered, and the nucleic acid sequence of interest can be recovered too.

Alternatively, according to another embodiment, reporter cells are present in the second droplet, instead of beads and reagents mixture. Said reporter cells are as described above for the cell sorting process. This would correspond to a functional assay: indeed, each well where the biological entity reacts with the reporter cell would be identified.

The barcoding process of the invention thus comprises:
- introducing emulsion droplets comprising cells into a microfluidic chip of the present invention, wherein each droplet comprises a single cell;
- putting a second droplet onto each emulsion droplet, wherein the second droplet comprises a defined number of beads coated with capture agents, preferably a single bead, and a reagents mixture, so that a fusion between each second droplet and each emulsion droplet is obtained, and nucleic acid sequences of interest are delivered in said fusion. Typically, if the nucleic acid of interest is present in a cell, it will link to the capture agent present on the bead; and

- capturing and optionally barcoding the nucleic acid sequences of interest;
- and then optionally recovering the fused droplets.

The process for manufacturing a microfluidic chip according to the invention will now be described in details.

"Sol-gel solution" is conventionally taken to mean a solution comprising one or more metal or metalloid molecular precursors and one or more organic solvents.

By "mold presenting a relief pattern and having a different thickness over the whole of the mold", it is meant a mold presenting a relief pattern which is typically complementary to nano- or microchannels (i.e. wherein the thickness is different over the whole of the mold). Preferably, the relief pattern of the mold comprises protrusions, so that the gelled sol-gel solution of step b) comprises microchannels or nanochannels. Said mold is called a master mold; it is a mold of the microfluidic chip to be prepared. Preferably, it is a mold presenting a relief pattern of 1 µm to 1 mm high. Preferably, the mold comprises a material of the family of epoxy-type negative near-UV photoresists or a polydimethylsiloxane material. One group of commonly used epoxy-type negative near-UV photoresist materials are octafunctional epoxidized novolac resins, and particularly photoresist materials based on the SU-8 family of photoresists made from Shell Chemical's EPON® SU-8 epoxy resin. Said commercial resin directly in solution and ready-to-use is sold by Microchem. The SU-8 family of photoresist materials is popular because it can provide relatively thick-film (usually from 500 nm to 1 mm) photoresist films at relatively low costs. In addition to being a relatively thick-film photoresist material, SU-8 is well suited for acting as a mold for electroplating because of its relatively high thermal stability (Tg greater than 200°C for the cross-linked or post-exposure photoresist material).

Typically, the process according to the present invention further comprises, before step a), a step of preparing the mold.

The protocol for preparing the mold may be as described in the document "SU-8 2000, Permanent Epoxy Negative Photoresist, processing guidelines" (available under the following link: http://www.microchem.com/pdf/SU-82000DataSheet2025thru2075Ver4.pdf).

Typically, it may be prepared by the following steps using the material of the family of epoxy-type negative near-UV photoresists (i.e. SU-8 family of photoresist materials):
- substrate pretreat: the substrate should be cleaned and dried prior to applying the photoresist. The substrate may typically be cleaned with a piranha wet etch (using H2SO4 & H2O2) followed by a deionized water rinse; or with reactive ion etching (RIE);
- coat: the substrate is coated with the photoresist under adequate spin conditions;
- edge bead removal (EBR): during the spin coat process step, a build-up of photoresist may occur on the edge of the substrate. In order to minimize contamination of the hotplate, this thick bead should be removed. This may be done by using a small stream of solvent (such as MicroChem's EBR PG) at the edge of the wafer either at the top or from the bottom;
- soft bake: the duration of convection oven baking (typically at 65°C and then 95°C) depends on the thickness to be obtained ;
- exposure: to obtain vertical sidewalls in the photoresist, a long pass filter may be used to eliminate UV radiation below 350 nm;
- post exposure bake (PEB): it generally takes place directly after exposure;
- development: development occurs using solvents such as ethyl lactate or diacetone alcohol;
- rinse and dry; and
- optionally hard bake or removal.

The mold which may be used may also be a PDMS mold. Such a material is more flexible and allows an easy peeling.

The process of the invention comprises successively:
a) casting a sol-gel solution made with tetraethyl orthosilicate onto a mold presenting a relief pattern and having a different thickness over the whole of the mold;
b) gelling the sol-gel solution;
c) unmolding and drying the gel obtained in b), so as to obtain a solid glass; and
d) bonding said solid glass to a support, so as to obtain the microfluidic chip.

Typically, before step a), the process of the invention comprises a step of preparing the sol-gel solution by a method comprising:
i) mixing tetraethyl orthosilicate (TEOS) with ethanol and water;
ii) adding hydrochloric acid to the mixture of step i). Preferably, the molar ratios of TEOS:ethanol:water:hydrochloric acid is around 0.8-1.2: 9-11: 2.5-4.5: 0.002-0.005;
iii) refluxing the mixture of step ii) at a temperature of at least 60°C for hydrolyzing tetraethyl orthosilicate. Typically, the temperature is comprised between 65°C and 100°C. preferably, step iii) lasts between 1 h and 2h;
iv) cooling the resulting mixture of step iii) at ambient temperature; and
v) adding an aqueous solution of ammonium hydroxide to the cooled mixture of step iv), and stirring to obtain the sol-gel solution.

The preparation of the sol-gel solution is typically described in Huang & Chou, Ceramics International 29 (2003), 485-493 (Studies on the spin coating process of silica films).

Steps i) to iii) above correspond to the "sol" synthesis, i.e. to a hydrolysis reaction.

Steps iv) and v) above correspond to the condensation-polymerization reaction.

The sol-gel solution prepared with TEOS is casted onto the required mold; this corresponds to step a). Said solution is still liquid.

The sol-gel solution prepared with TEOS is casted onto a mold of the object to be manufactured, intended, after gelling and drying, to constitute the microfluidic chip. This casting step, or pouring step, is thus different from an injection step.

Then, said sol-gel solution is gelled (step b).

The gel obtained in b) is then unmolded and dried; this is step c). The drying step may be performed according to two different ways:
- evaporative drying: the gel obtained in b), once unmolded, is dried 2 to 3 weeks under a controlled atmosphere (e.g. nitrogen); or
- supercritical drying: the gel obtained in b) is put in a sealed chamber and under pressure. Carbon dioxide is then added, and forms a supercritical fluid (i.e. which is liquid and gaseous at the same time). It prevents constraints to appear in the material, and thus prevents cracks to happen.

At the end of step c), a solid glass is obtained.

Then, said solid glass is bonded to a support, so as to obtain the microfluidic chip; this is step d).

Preferably, the support used in step d) is a glass slide, or a substrate made up of a material which is coated with a SiO2 layer using a sputtering technique.

Preferably, step d) comprises anodic bonding for glass or SiO2 coated substrates, or plasma bonding or thermal bonding.

Preferably, for the cell sorter and cell sorting process, step d) comprises anodic bonding for SiO2 coated substrates, performed with a piezoelectric material, such as lithium niobate or lithium tantalate substrate. Preferably, said step d) is performed at a temperature comprised between room temperature (around 20°C) and 400°C.

The term "anodic bonding" is used to identify the bonding of the solid glass substrate of the invention, to glass or silicon supports, preferably SiO2 coated substrates, with a high content of alkali oxides.

The bonding occurs when the two substrates are heated after being brought in contact and an electric field is applied.

During anodic bonding, oxides dissociate and the mobile alkali ions are driven by the electric field into the glass, creating an oxygen rich layer notably at the silicon-glass interface. Oxygen ions are driven by the electric field to the silicon surface and produce oxidation of Si. The resulting bond strength is very high and the process is irreversible.

The present invention also relates to a microfluidic chip obtainable by the process according to the invention.

Said microfluidic chip may be used for screening proteins, preferably antibodies. Preferably, said microfluidic chip is a static droplet array or a droplet sorter, preferably a surface acoustic wave droplet sorter.

Finally, the present invention also relates to a process for screening cells producing a protein of interest, preferably immune cells, comprising the following steps:
- introducing emulsion droplets comprising cells producing a protein, preferably immune cells, and either beads coated with capture agents or cells expressing capture agents at their surface or within the cell, into a microfluidic chip of the present invention;
- incubating the resulting chip under conditions allowing protein secretion if necessary; and
- sorting cells producing the protein of interest based on a specific optical signal.

Said signal may be fluorescent or luminescent. It may be detected for example after a laser excitation, with a suitable bandpass filter such as a photomultiplier or an avalanche photodiode.

Preferably, each emulsion droplet comprises (i) a single cell, preferably a single immune cell and more preferably a single antibody- or cytokine-producing cell, and (ii) preferably a single bead coated with capture agents or a suspension of magnetic beads coated with capture agents, or a single cell expressing capture agents.

Alternatively, the present invention also relates to a process for identifying nucleic acid sequences of interest, comprising the following steps:
- introducing emulsion droplets comprising cells into a microfluidic chip of the present invention, wherein each droplet comprises a single cell;
- putting a second droplet onto each emulsion droplet, wherein the second droplet comprises a defined number of beads coated with capture agents, preferably a single bead, and a reagents mixture, so that a fusion between each second droplet and each emulsion droplet is obtained, and nucleic acid sequences of interest are delivered in said fusion. The fusion may be obtained by applying a voltage; and
- capturing and optionally barcoding the nucleic acid sequences of interest.

## Claims

1. A process for manufacturing a microfluidic chip comprising a solid material obtained from a sol-gel solution, the process comprising successively:
a) casting a sol-gel solution made with tetraethyl orthosilicate onto a mold presenting a relief pattern and having a different thickness over the whole of the mold;
b) gelling the sol-gel solution;
c) unmolding and drying the gel obtained in b), so as to obtain a solid glass; and
d) bonding said solid glass to a support, so as to obtain the microfluidic chip.

2. The process according to claim 1, wherein the mold comprises a material of the family of epoxy-type negative near-UV photoresists or a polydimethylsiloxane material.

3. The process according to any one of claims 1 to 2, further comprising, before step a), a step of preparing the mold.

4. The process according to any one of claims 1 to 3, wherein the relief pattern of the mold comprises protrusions, so that the gelled sol-gel solution of step b) comprises microchannels or nanochannels.

5. The process according to any one of claims 1 to 4, wherein the support used in step d) is a glass slide or a substrate made up of a material which is coated with a SiO2 layer using a sputtering technique.

6. The process according to any one of claims 1 to 5, wherein step d) comprises anodic bonding for glass or SiO2 coated substrates, or plasma bonding or thermal bonding.

7. The process according to claim 6, wherein anodic bonding for SiO2 coated substrates is performed with a piezoelectric material, such as lithium niobate or lithium tantalate substrate.

8. The process according to any one of claims 1 to 7, wherein said process further comprises, before step a), a step of preparing the sol-gel solution by a method comprising:
i) mixing tetraethyl orthosilicate with ethanol and water;
ii) adding hydrochloric acid to the mixture of step i);
iii) refluxing the mixture of step ii) at a temperature of at least 60°C for hydrolyzing tetraethyl orthosilicate;
iv) cooling the resulting mixture of step iii) at ambient temperature;
v) adding an aqueous solution of ammonium hydroxide to the cooled mixture of step iv), and stirring to obtain the sol-gel solution.

9. Microfluidic chip obtainable by the process according to any one of claims 1 to 8.

10. Use of a microfluidic chip according to claim 9 for screening proteins, preferably antibodies.

11. Use according to claim 10, wherein the microfluidic chip is a static droplet array or a droplet sorter, preferably a surface acoustic wave droplet sorter.

12. A process for screening cells producing a protein of interest, preferably immune cells, comprising the following steps:
- introducing emulsion droplets comprising cells producing a protein, and either beads coated with capture agents or cells expressing capture agents on their surface or within the cell, said emulsion droplets being optionally previously incubated in bulk, into a microfluidic chip according to claim 9; and
- sorting cells producing the protein of interest based on a specific optical signal.

13. A process for screening cells producing a protein of interest, preferably immune cells, comprising the following steps:
- introducing emulsion droplets comprising cells producing a protein, and either beads coated with capture agents or cells expressing capture agents at their surface or within the cell, into a microfluidic chip according to claim 9;
- incubating the resulting chip under conditions allowing protein secretion; and
- sorting cells producing the protein of interest based on a specific optical signal.

14. A process according to claim 12 or 13, wherein each emulsion droplet comprises (i) a single cell, and (ii) a single bead coated with capture agents or a suspension of magnetic beads coated with capture agents, or a single cell expressing capture agents.

15. A process for identifying nucleic acid sequences of interest, comprising the following steps:
- introducing emulsion droplets comprising cells into a microfluidic chip according to claim 9, wherein each droplet comprises a single cell;
- putting a second droplet onto each emulsion droplet, wherein the second droplet comprises a defined number of beads coated with capture agents, preferably a single bead, and a reagents mixture, so that a fusion between each second droplet and each emulsion droplet is obtained, and nucleic acid sequences of interest are delivered in said fusion; and
- capturing and optionally barcoding the nucleic acid sequences of interest,
- and then optionally recovering the fused droplets.
